(19) **Europäisches Patentamt European Patent Office Office européen des brevets**

(11) **EP 3 399 314 B1**

(12) **EUROPEAN PATENT SPECIFICATION**

(45) Date of publication and mention of the grant of the patent:
**07.02.2024 Bulletin 2024/06**

(21) Application number: **16881790.6**

(22) Date of filing: **27.12.2016**

(51) International Patent Classification (IPC):
**G01N 33/574** *(2006.01)*   **C07K 14/47** *(2006.01)*
**G16H 50/20** *(2018.01)*   **C07K 16/28** *(2006.01)*

(52) Cooperative Patent Classification (CPC):
**G01N 33/574; C07K 14/47; C07K 16/2818; C07K 16/2827; G01N 33/57423; G01N 33/5743; G01N 33/57438; G01N 33/57449; G01N 33/57488; G16H 50/20;** G01N 2333/70521; G01N 2333/70532; G01N 2333/70596

(86) International application number:
**PCT/JP2016/088978**

(87) International publication number:
**WO 2017/115816 (06.07.2017 Gazette 2017/27)**

(54) **METHOD FOR DETERMINING CANCER, AND DEVICE AND COMPUTER PROGRAM FOR DETERMINING CANCER**

VERFAHREN ZUR BESTIMMUNG VON KREBS SOWIE VORRICHTUNG UND COMPUTERPROGRAMM ZUR BESTIMMUNG VON KREBS

PROCÉDÉ DE DÉTECTION D'UN CANCER, ET DISPOSITIF ET PROGRAMME INFORMATIQUE DE DÉTECTION D'UN CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2015 JP 2015256605**

(43) Date of publication of application:
**07.11.2018 Bulletin 2018/45**

(73) Proprietors:
- **Kyoto University**
  **Kyoto-shi, Kyoto 606-8501 (JP)**
- **Sysmex Corporation**
  **Kobe-shi, Hyogo 651-0073 (JP)**

(72) Inventors:
- **HONJO, Tasuku**
  **Kyoto-shi**
  **Kyoto 606-8501 (JP)**
- **CHAMOTO, Kenji**
  **Kyoto-shi**
  **Kyoto 606-8501 (JP)**
- **GOTO, Megumi**
  **Kobe-shi**
  **Hyogo 651-0073 (JP)**
- **UGA, Hitoshi**
  **Kobe-shi**
  **Hyogo 651-0073 (JP)**

(74) Representative: **De Clercq & Partners**
  **Edgard Gevaertdreef 10a**
  **9830 Sint-Martens-Latem (BE)**

(56) References cited:
**WO-A2-2015/184061**

- **Yasunori Akutsu: "SERUM LEVEL OF PD-1, PD-L1, AND PD-L2 AS A NEW BIOMARKER FOR ESOPHAGEAL CANCER", The American Journal of GASTROENTEROLOGY, 1622, 1 October 2015 (2015-10-01), page S694, XP055398973, Retrieved from the Internet: URL:http://www.nature.com/ajg/journal/v110 /n1s/pdf/ajg2015274a.pdf [retrieved on 2017-08-16]**

- Ouyang Shaobo ET AL: "LEVELS OF PROGRAMMED DEATH-1 AND PROGRAMMED DEATH LIGAND-1 IN THE PERIPHERAL BLOOD OF PATIENTS WITH ORAL SQUAMOUS CELL CARCINOMA AND ITS CLINICAL IMPLICATIONS", WEST CHINA JOURNAL OF STOMATOLOGY, 1 October 2015 (2015-10-01), pages 529-533, XP055399016, DOI: 10.7518/hxkq.2015.05.019 Retrieved from the Internet: URL:http://www.hxkqyxzz.net/fileup/PDF/201505019.pdf [retrieved on 2017-08-16]
- JAN TROST JORGENSEN: "Companion diagnostic assays for PD-1/PD-L1 checkpoint inhibitors in NSCLC", EXPERT REVIEW OF MOLECULAR DIAGNOSTICS, vol. 16, no. 2, 27 November 2015 (2015-11-27), pages 131-133, XP055426614, GB ISSN: 1473-7159, DOI: 10.1586/14737159.2016.1117389
- AKUTSU , YASUNORI: "1622 SERUM LEVEL OF PD-1, PD-L1, AND PD-L2 AS A NEW BIOMARKER FOR ESOPHAGEAL CANCER", AMERICAN JOURNAL OF GASTROENTEROLOGY, vol. 110, no. Suppl. 1, October 2015 (2015-10), page 694, XP055398973, ISSN: 0002-9270
- ZHANG PENG: "LEVELS OF PROGRAMMED DEATH-1 AND PROGRAMMED DEATH LIGAND-1 IN THE PERIPHERAL BLOOD OF PATIENTS WITH ORAL SQUAMOUS CELL CARCINOMA AND ITS CLINICAL IMPLICATIONS", WEST CHINA JOURNAL OF STOMATOLOGY, vol. 33, no. 5, October 2015 (2015-10), pages 529-533, XP055399016, ISSN: 1000-1182
- LAURENT STEFANIA: "THE ENGAGEMENT OF CTLA-4 ON PRIMARY MELANOMA CELL LINES INDUCES ANTIBODY- DEPENDENT CELLULAR CYTOTOXICITY AND TNF-alpha PRODUCTION", JOURNAL OF TRANSLATIONAL MEDICINE, vol. 11 , XP055100523, ISSN: 1479-5876
- MASUDA AKINORI: "CLINICAL SIGNIFICANCE OF SERUM SOLUBLE T CELL REGULATORY MOLECULES IN CLEAR CELL RENAL CELL CARCINOMA", BIOMED RESEARCH INTERNATIONAL, vol. 2014, 2014, pages 1-6, XP055399021, ISSN: 2314-6141
- DATABASE CAPLUS retrieved from STN Database accession no. 2012:1501792 & ILHAN, F.: "SERUM AND PLEURAL FLUID SOLUBLE CTLA-4 LEVELS ARE INCREASED IN LUNG CANCER PATIENTS", NOVEL SCIENCE INTERNATIONAL JOURNAL OF MEDICAL SCIENCES, vol. 1, no. 5 2012, pages 145-147, ISSN: 2278-0025
- ERFANI NASROLLAH: "CIRCULATING SOLUBLE CTLA4 (SCTLA4) IS ELEVATED IN PATIENTS WITH BREAST CANCER", CANCER INVESTIGATION, vol. 28, no. 8, October 2010 (2010-10), pages 828-832, XP009511386, ISSN: 1532-4192
- MANSOUR , AMANY: "INCREASED EXPRESSION OF COSTIMULATORY MOLECULES CD 86 AND SCTLA-4 IN PATIENTS WITH ACUTE LYMPHOBLASTIC LEUKEMIA", LEUKEMIA & LYMPHOMA, vol. 55, no. 9, 2014, pages 2120-2124, XP009511379, ISSN: 1029-2403
- KUCUKHUSEYIN, OZLEM: "INDIVIDUAL AND COMBINED EFFECTS OF CTLA4- CD 28 VARIANTS AND OXIDANT- ANTIOXIDANT STATUS ON THE DEVELOPMENT OF COLORECTAL CANCER", ANTICANCER RESEARCH, vol. 35, no. 10, October 2015 (2015-10), pages 5391-5400, XP055399033, ISSN: 0250-7005
- SUNAKAWA MIKA: "Clinical impact of soluble B7- HI (PD-L1) in multiple myeloma", The Japanese Journal of Clinical Hematology, vol. 56, no. 9, 30 September 2015 (2015-09-30), page 1611, XP009511381, ISSN: 0485-1439
- CHEN YONGJING: "DEVELOPMENT OF A SANDWICH ELISA FOR EVALUATING SOLUBLE PD-L1 ( CD 274) IN HUMAN SERA OF DIFFERENT AGES AS WELL AS SUPERNATANTS OF PD-L1+ CELL LINES", CYTOKINE, vol. 56, no. 2, November 2011 (2011-11), pages 231-238, XP028301724, ISSN: 1043-4666
- HISAE IINUMA: "Ko PD-1 Kotaiyaku no Biomarker -Kessho PD-L1 Tanpaku no Biomarker to shite no Kanosei", Japanese Journal of Cancer and Chemotherapy, vol. 43, no. 9, September 2016 (2016-09), pages 1030-1035, XP009511384, ISSN: 0385-0684
- OKUMA Y: "HIGH PLASMA LEVELS OF SOLUBLE PROGRAMMED CELL DEATH LIGAND 1 ARE PROGNOSTIC FOR REDUCED SURVIVAL IN ADVANCED LUNG CANCER", Lung Cancer, vol. 104, 5 December 2016 (2016-12-05), pages 1-6, XP029923782, ISSN: 0169-5002
- Zhang Jie ET AL: "Circulating PD-L1 in NSCLC patients and the correlation between the level of PD-L1 expression and the clinical characteristics", Thoracic Cancer, vol. 6, no. 4, 5 March 2015 (2015-03-05), pages 534-538, XP55891266, ISSN: 1759-7706, DOI: 10.1111/1759-7714.12247 Retrieved from the Internet: URL:https://onlinelibrary.wiley.com/doi/full-xml/10.1111/1759-7714.12247>

**Description**

TECHNICAL FIELD

The present invention relates to a method for determining lung cancer, kidney cancer, ovarian cancer, or melanoma. Furthermore, the present invention relates to an apparatus and computer program for determining lung cancer, kidney cancer, ovarian cancer, or melanoma.

BACKGROUND ART

Receptor molecules called PD-1 (Programmed cell death-1) are expressed on the surface of activated T cells (see Non Patent Literature 1). PD-1 is known as a molecule that suppresses excessive activation of T cells and negatively regulates the immune response of a living body. In addition, on the surface of activated T cells, molecules called CTLA-4 (Cytotoxic T lymphocyte antigen-4) are also expressed. Similarly to PD-1, CTLA-4 also has a function of suppressing activation of T cells. On the other hand, some cells in the cancer section express PD-L1 (Programmed cell death-ligand 1), PD-1 ligand, on the cell surface. It is known that such cells in the cancer section suppress the activation of T cells through the binding of PD-L1 of their own to PD-1 of T cells, and avoid T cell attack. As a result, cancer increases in vivo. In fact, there is a correlation between PD-L1 expression level in the cancer section and poor prognosis rate of cancer patients. Zhang et al. (2015, see Non Patent Literature 2) evaluated the association between circulating PD-L1 expression and clinical characteristics in patients with advanced non-small cell lung cancer (NSCLC). They found that the expression of PL-L1 in advanced NSCLC patients was significantly upregulated compared with the healthy control, and that a high PD-L1 expression had a worse prognosis than a low expression in patients. As described above, PD-L1, PD-1 and CTLA-4 are also referred to as immune checkpoint molecules because they are involved in suppression of immunity.

CITATIONS LIST

Non Patent Literature

Non Patent Literature 1: Agata Y. et al., Expression of the PD-1 antigen on the surface of stimulated mouse T and B lymphocytes. Int. Immunol., Vol. 8, pp. 765-772 (1996)
Non Patent Literature 2: Zhang et al. Circulating PD-L1 in NSCLC patients and the correlation between the level of PD-L1 expression and the clinical characteristics. Thoracic Cancer, Vol. 6, pp. 534-538 (2015)

SUMMARY OF INVENTION

TECHNICAL PROBLEMS

Since PD-L1 is a molecule present on the cell membrane, in order to determine cancer by PD-L1 detection, it is necessary to use the tissue collected from a subject as a sample. For example, a method of collecting a tumor tissue from a cancer patient to prepare a pathological tissue specimen and performing immunostaining of PD-L1 is known. In addition, a method of determining immunogenicity by confirming gene mutation in cancer cells is also adopted. However, due to the following reasons, the conventional method has some points to be improved. 1) Collection of tissues imposes a heavy burden on subjects. 2) When the cause of cancer onset is in T cells, the result cannot be obtained by the determination method based on the detection of PD-L1. 3) Tissue specimens vary widely depending on the collection site, and the expression level of immune checkpoint-related molecules in whole cancer tissue or whole body cannot be quantified. Therefore, it is desirable to have a cancer determination method that quantifies the amount of immune checkpoint-related molecules of cancer cells and T cells systemically, with a small burden on subjects.

SOLUTIONS TO PROBLEMS

As described above, PD-L1, PD-1 and CTLA-4 are molecules involved in suppression of immune activation state, and it is known that, when the immune state of the living body is activated, expression of these molecules on the cell surface increases. Here, when the living body is affected by cancer, it is considered that T cells are activated to exclude cancer in the living body, and expression of CTLA-4 and PD-1 increases on the T cell surface. Moreover, on the cell surface of the cancer section, it is considered that the expression of PD-L1 increases to avoid attack of activated T cells. The present inventors have considered that, as the expression of these molecules increases on the cell surface, the amount of molecules released from the cell surface also increases. That is, the present inventors have considered that PD-L1, PD-1 and CTLA-4 released from the cell surface increase in the body of a cancer patient.

The present inventors have found that, by measuring free PD-L1 and free PD-1 as free protein markers in a liquid sample such as a blood sample, whether the sample is a sample collected from a subject suffering from lung cancer, kidney cancer, ovarian cancer, or melanoma can be discriminated, thereby completing the present invention.

Therefore, the present invention provides a method for determining cancer, wherein

the cancer is lung cancer, kidney cancer, ovarian cancer, or melanoma, the method including the steps of: measuring free PD-L1 and free PD-1 as free protein markers in a liquid sample collected from a subject, and determining whether or not the liquid sample is a sample collected from a subject suffering from cancer, wherein in the determination step, measured values of the free protein markers acquired in the measurement step are compared with a predetermined threshold value corresponding to each of the markers, and whether or not the liquid sample is a sample collected from a subject suffering from the cancer is determined, based on the comparison result or wherein in the determination step, a predicted value acquired by multivariate analysis from measured values of the free protein markers acquired in the measurement step is compared with a predetermined threshold value corresponding to the predicted value, and whether or not the liquid sample is a sample collected from a subject suffering from the cancer is determined, based on the comparison result.

Also, the present invention provides a cancer determination apparatus, wherein the cancer is lung cancer, kidney cancer, ovarian cancer, or melanoma, comprising a computer containing a processor and a memory under control of the processor, in which a computer program for making the computer execute the steps of acquiring measured values of free PD-L1 and free PD-1 as free protein markers in a liquid sample collected from a subject, and determining whether or not the liquid sample is a sample collected from a subject suffering from cancer by comparing the measured values of the free protein markers with a predetermined threshold value corresponding to each of the markers, and determining whether or not the liquid sample is a sample collected from a subject suffering from the cancer based on the comparison result or
by comparing a predicted value acquired by multivariate analysis from measured values of the free protein markers with a predetermined threshold value corresponding to the predicted value, and determining whether or not the liquid sample is a sample collected from a subject suffering from the cancer based on the comparison result, is recorded in the memory.

Furthermore, the present invention provides a computer program for determining cancer which is recorded on a computer readable medium, in which the computer program is a computer program for making the computer execute the steps of acquiring measured values of free PD-L1 and free PD-1 as free protein markers in a liquid sample collected from a subject, and determining whether or not the liquid sample is a sample collected from a subject suffering from cancer by comparing the measured values of the free protein markers with a predetermined threshold value corresponding to each of the markers, and determining whether or not the liquid sample is a sample collected from a subject suffering from the cancer based on the comparison result or
by comparing a predicted value acquired by multivariate analysis from measured values of the free protein markers with a predetermined threshold value corresponding to the predicted value, and determining whether or not the liquid sample is a sample collected from a subject suffering from the cancer based on the comparison result, and wherein the cancer is lung cancer, kidney cancer, ovarian cancer, or melanoma.

ADVANTAGEOUS EFFECTS OF INVENTION

According to the present invention, since a liquid sample collected from a subject is used, the burden on the subject in collecting the sample is small. In addition, the present invention makes it possible to objectively evaluate whether or not the liquid sample is a sample collected from a subject suffering from lung cancer, kidney cancer, ovarian cancer, or melanoma.

BRIEF DESCRIPTION OF DRAWINGS

Fig. 1 is a schematic diagram showing an example of a cancer determination apparatus.
Fig. 2 is a block diagram showing a hardware configuration of a cancer determination apparatus.
Fig. 3A is a flowchart for a cancer determination using a cancer determination apparatus.
Fig. 3B is a flowchart for a cancer determination using a cancer determination apparatus.
Fig. 4 is a flowchart for a cancer determination using a cancer determination apparatus.

Fig. 5 is an ROC curve when a determination is made from predicted values based on concentration values of free PD-L1.

Fig. 6 is an ROC curve when a determination is made from predicted values based on concentration values of free PD-1.

Fig. 7 is an ROC curve when a determination is made from predicted values based on concentration values of free CTLA-4.

Fig. 8 is an ROC curve when a determination is made from predicted values based on concentration values of free PD-L1 and free PD-1.

Fig. 9 is an ROC curve when a determination is made from predicted values based on concentration values of free PD-L1, free PD-1 and free CTLA-4.

DESCRIPTION OF EMBODIMENTS

[1. Cancer Determination Method]

In the cancer determination method of the present invention (hereinafter also simply referred to as "determination method"), first, free PD-L1 and free PD-1 are measured as free protein markers in a liquid sample collected from a subject. In particular embodiments, in addition to free PD-L1 and free PD-1, free CTLA-4 may be further measured.

The subject from which the liquid sample is collected is not particularly limited, and may be a person suspected of suffering from cancer, for example. The type of cancer is lung cancer, kidney cancer, ovarian cancer or melanoma.

The liquid sample is not particularly limited as long as it is a liquid sample that has been collected from a subject and can contain free protein. Examples of such liquid sample include a blood sample, cerebrospinal fluid, pleural effusion, ascites, lymph, urine, and the like. In embodiments, a blood sample is preferable as the liquid sample. Examples of the blood sample include whole blood, plasma and serum, and plasma and serum are particularly preferable.

When insoluble contaminants such as cells are contained in the liquid sample, for example, impurities may be removed from the liquid sample by a known means such as centrifugal separation and filtration. Further, the liquid sample may be diluted with an appropriate aqueous medium as necessary. Such an aqueous medium is not particularly limited as long as it does not interfere with the measurement described later, and examples thereof include water, physiological saline, a buffer solution, and the like. The buffer solution is not particularly limited as long as it has a buffering effect at a pH near neutrality (for example, a pH of 6 or more and 8 or less). Examples of the buffer solution include Good buffers such as HEPES, MES, Tris and PIPES, phosphate buffered saline (PBS), and the like.

Free PD-L1, free PD-1 and free CTLA-4 measured as markers in the present method are all free proteins. As used herein, the term "free protein" refers to a protein that is detached from the surface of a cell in a liquid sample and is present outside the cell. Such a free protein may be either a protein solubilized in a liquid component (liquid phase) of the liquid sample, a protein encapsulated in a vesicle, or a protein present on the surface of a vesicle. The vesicle is not particularly limited as long as it is a small vesicle composed of a membrane. The vesicle may contain a liquid phase therein. Preferred examples of the vesicles include extracellular vesicles such as exosomes, microvesicles, apoptotic bodies, and the like.

The means for measuring a free protein marker is not particularly limited as long as it can acquire a value reflecting the amount or concentration of the free protein marker contained in the liquid sample (hereinafter also referred to as a "measured value of the marker"). In embodiments, a method of capturing a free protein marker using a substance capable of specifically binding to the marker is preferable. The free protein marker contained in the liquid sample can be measured by detecting the free protein marker captured by such a substance by a method known in the art.

Examples of the substance capable of specifically binding to a free protein marker include an antibody, an aptamer and the like, among which an antibody is particularly preferable. Antibodies themselves against each of the PD-L1, PD-1 and CTLA-4 proteins are known in the art and are generally available. An antibody against a free protein marker is not particularly limited as long as it is an antibody capable of specifically binding to a free protein marker. Such an antibody may be any of monoclonal antibodies, polyclonal antibodies, and fragments thereof (for example, Fab, F(ab')2, etc.). Alternatively, a commercially available antibody may be used.

A method for measuring a free protein marker using an antibody is not particularly limited and can be appropriately selected from known immunological measurement methods. In particular embodiments, an enzyme-linked immunosorbent assay (ELISA method) is preferred, and a sandwich ELISA method being particularly preferred. As an example of the measurement step, the case of measuring a free protein marker in a liquid sample by a sandwich ELISA method will be described below.

First, a complex containing a free protein marker and an antibody for capturing the free protein marker (hereinafter also referred to as "capture antibody") and an antibody for detecting the free protein marker (hereinafter also referred to as "detection antibody") is formed on a solid phase. The complex can be formed by mixing a liquid sample that can contain a free protein marker, a capture antibody, and a detection antibody. Then, a solution containing the complex is brought into contact with a solid phase capable of capturing the capture antibody, whereby the complex can be formed on the solid phase. Alternatively, a solid phase preliminarily immobilized with the capture antibody may be used. That is, a solid phase immobilized with the capture antibody, the liquid sample, and the detection antibody are brought into contact with each other, whereby the complex can be formed on the solid phase. When both the capture antibody and the detection antibody are monoclonal antibodies, it is preferable that the epitopes be different from each other.

The mode of immobilization of the capture antibody on the solid phase is not particularly limited. For example, the capture antibody and the solid phase may be bound directly, or the capture antibody and the solid phase may be indirectly bound via another substance. Examples of the direct bond include physical adsorption and the like. Examples of the indirect bond include a bond via a combination of biotin and avidin or streptavidin (hereinafter also referred to as "avidins"). In this case, by preliminarily modifying the capture antibody with biotin and previously binding avidins to the solid phase, the capture antibody and the solid phase can be indirectly bound via the bond between the biotin and the avidins.

The material of the solid phase is not particularly limited, and it can be selected from, for example, organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, etc.), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include particles, membranes, microplates, microtubes, test tubes, and the like. Among them, particles are preferable, and magnetic particles are particularly preferable.

In particular embodiments, B/F (Bound/Free) separation for removing an unreacted free component not forming a complex may be performed between the process of forming the complex and the process of detecting the complex. The unreacted free component refers to a component not constituting a complex. Examples thereof include capture antibodies not bound to the free protein marker, detection antibodies, and the like. The means of B/F separation is not particularly limited, and when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particles with a magnet, which is preferable from the viewpoint of automation. After removing the unreacted free component, the solid phase capturing the complex may be washed with a suitable aqueous medium such as PBS.

Then, the complex formed on the solid phase is detected by a method known in the art, whereby the measured value of the free protein marker contained in the liquid sample can be acquired. For example, when an antibody labeled with a labeling substance is used as a detection antibody, the measured value of the marker in the liquid sample can be acquired by detecting a signal generated by the labeling substance. Alternatively, also when a labeled secondary antibody against the detection antibody is used, the measured value of the marker in the liquid sample can be acquired in the same manner.

Also, as an example of a method for measuring a free protein marker using an antibody, the immune complex transfer method described in JP H01-254868 A can be used.

The phrase "detecting a signal" herein includes qualitatively detecting the presence or absence of a signal, quantifying a signal intensity, and semi-quantitatively detecting the intensity of a signal. Semi-quantitative detection means to show the intensity of the signal in stages such as "no signal generated", "weak", "medium", "strong", and the like. It is preferable to detect the intensity of a signal quantitatively or semi-quantitatively.

The labeling substance is not particularly limited as long as a detectable signal is generated. For example, it may be a substance which itself generates a signal (hereinafter also referred to as "signal generating substance") or a substance which catalyzes the reaction of other substances to generate a signal. Examples of the signal generating substance include fluorescent substances, radioactive isotopes, and the like. Examples of the substance that catalyzes the reaction of other substances to generate a detectable signal include enzymes. Examples of the enzymes include alkaline phosphatase, peroxidase, β-galactosidase, luciferase, and the like. Examples of the fluorescent substance include fluorescent dyes such as fluorescein isothiocyanate (FITC), rhodamine and Alexa Fluor (registered trademark), fluorescent proteins such as GFP, and the like. Examples of the radioisotopes include $^{125}$I, $^{14}$C, $^{32}$P, and the like. Among them, an enzyme is preferable as a labeling substance, and alkaline phosphatase and peroxidase are particularly preferable.

Methods for detecting a signal themselves are known in the art. A measurement method according to the type of signal derived from the labeling substance may be appropriately selected. For example, when the labeling substance is an enzyme, signals such as light and color generated by reacting a substrate for the enzyme can be measured by using a known apparatus such as a spectrophotometer.

The substrate of the enzyme can be appropriately selected from known substrates according to the type of the enzyme. For example, when alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate. Also, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB).

When the labeling substance is a radioactive isotope, radiation as a signal can be measured using a known apparatus such as a scintillation counter. Also, when the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used.

The detection result of the signal can be used as the measured value of the marker. For example, when quantitatively detecting the intensity of a signal, the signal intensity value itself or a value acquired from the measured value can be used as the measured value of the marker. Examples of the value acquired from the measured value of the signal intensity include a value acquired by subtracting the measured value of a negative control sample or the background value from the measured value, a value acquired by applying the measured value to a calibration curve, and the like. The negative control sample can be appropriately selected, and examples thereof include a liquid sample obtained from a healthy subject and the like.

In particular embodiments, it is preferable to measure the free protein marker contained in the liquid sample by a sandwich ELISA method using a capture antibody immobilized on magnetic particles and a detection antibody labeled with a labeling substance. In this case, measurement may be carried out using a commercially available fully automated immunoassay system such as HISCL series (manufactured by Sysmex Corporation).

Next, in the sample determination method according to the present invention, whether or not the liquid sample is a sample collected from a subject suffering from lung cancer, kidney cancer, ovarian cancer, or melanoma is discriminated by comparing the measured values of the free protein markers with a predetermined threshold value corresponding to each of the markers, and determining whether or not the liquid sample is a sample collected from a subject suffering from the cancer based on the comparison result or

by comparing a predicted value acquired by multivariate analysis from measured values of the free protein markers with a predetermined threshold value corresponding to the predicted value, and determining whether or not the liquid sample is a sample collected from a subject suffering from the cancer based on the comparison result.

The measured values of the free protein markers acquired by the measurement are compared with a predetermined threshold value corresponding to each marker, and whether or not the liquid sample is a sample collected from a subject suffering from the cancer is determined, based on the comparison result.

For example, when the measured values of free PD-L1 and free PD-1 are acquired, the measured value of free PD-L1 is compared with the first threshold value, and the measured value of free PD-1 is compared with the second threshold value, then a determination is made based on the comparison results. As an example of the determination, when the measured value of free PD-L1 is higher than the first threshold value or is equal to the first threshold value and the measured value of free PD-1 is higher than the second threshold value or is equal to the second threshold value, it may be determined that the liquid sample is a sample collected from a subject suffering from the cancer. Also, when the measured value of free PD-L1 is lower than the first threshold value or the measured value of free PD-1 is lower than the second threshold value, it may be determined that the liquid sample is not one collected from a subject suffering from the cancer.

For example, when the measured values of free PD-L1, free PD-1 and free CTLA-4 are acquired, the measured value of free PD-L1 is compared with the first threshold value, the measured value of free PD-1 is compared with the second threshold value, and the measured value of free CTLA-4 is compared with the third threshold value, then a determination is made based on the comparison results. As an example of the determination, when the measured value of free PD-L1 is higher than the first threshold value or is equal to the first threshold value, the measured value of free PD-1 is higher than the second threshold value or is equal to the second threshold value, and the measured value of free CTLA-4 is higher than the third threshold value or is equal to the third threshold value, it may be determined that the liquid sample is a sample collected from a subject suffering from the cancer. Also, when the measured value of free PD-L1 is lower than the first threshold value, the measured value of free PD-1 is lower than the second threshold value, or the measured value of free CTLA-4 is lower than the third threshold value, it may be determined that the liquid sample is not one collected from a subject suffering from the cancer.

Alternatively, a predicted value acquired by multivariate analysis from the measured value of the free protein markers acquired by the measurement is compared with a predetermined threshold value corresponding to the predicted value, and whether or not the liquid sample is a sample collected from a subject suffering from the cancer may be determined, based on the comparison result. Multivariate analysis can be appropriately selected from known analytical methods such as logistic regression analysis, multiple regression analysis, and discriminant analysis. Logistic regression analysis is preferable among them.

For example, when the measured values of free PD-L1 and free PD-1 are acquired, these measured values are substituted into the discriminant created by multivariate analysis (for example, logistic regression analysis) to acquire a predicted value. Even when the measured value of free CTLA-4 is further acquired, the measured values of all the acquired markers are substituted into the discriminant created by multivariate analysis (for example, logistic regression analysis) in the same manner as above, to acquire a predicted value. Then, the acquired predicted value is compared with a predetermined threshold value, and a determination is made based on the comparison results. As an example of the determination, when the acquired predicted value is higher than the predetermined threshold value or is equal to the predetermined threshold value, it may be determined that the liquid sample is a sample collected from a subject suffering from the cancer. Further, when the acquired predicted value is lower than the predetermined threshold value, it may be determined that the liquid sample is not one collected from a subject suffering from the cancer.

In embodiments, when the measured values of free PD-L1 and free PD-1 are acquired, the predicted value may be calculated using a regression equation of the following formula (I). In addition, when the measured values of free PD-L1, free PD-1 and free CTLA-4 are acquired, the predicted value may be calculated using a regression equation of the following formula (II). These regression equations are equations constructed based on the multiple logistic model. In the formula, [PD-L1], [PD-1] and [CTLA-4] are the measured values of free PD-L1, free PD-1 and free CTLA-4, respectively. $b_0$ is a coefficient of the intercept, and $b_1$ to $b_3$ are coefficients of each free protein marker. The coefficients can be determined by a known method such as a maximum likelihood method.

[Expression 1]

$$P = \frac{1}{1 + \exp[-(b_0 + b_1 \times [\text{PD-L1}] + b_2 \times [\text{PD-1}])]} \quad (\text{I})$$

[Expression 2]

$$P = \frac{1}{1 + \exp[-(b_0 + b_1 \times [\text{PD-L1}] + b_2 \times [\text{PD-1}] + b_3 \times [\text{CTLA-4}])]} \quad (\text{II})$$

The predetermined threshold value corresponding to each marker and the threshold value corresponding to the predicted value described above are not particularly limited and can be set empirically, for example, by accumulating data of free protein markers in liquid samples collected from various cancer patients. Alternatively, the predetermined cut-off value may be set as follows. Liquid samples are collected from a healthy subject and various cancer patients, and measured values of free protein markers are acquired. Then, from the acquired measured values, values that can distinguish between the healthy subject and the cancer patient for each marker are obtained, and the obtained values are set as predetermined cut-off values corresponding to each marker. Alternatively, the measured values of the markers acquired from liquid samples of a healthy subject and various cancer patients are substituted into the discriminant created by multivariate analysis to acquire a predicted value of the healthy subject and a predicted value of the cancer patient. Then, based on the acquired predicted value, a value that can distinguish between the healthy subject and the cancer patient is obtained, and the obtained value is set as a predetermined cut-off value corresponding to the predicted value.

The determination method of the present invention makes it possible to provide a doctor and the like with information that assists diagnosis of lung cancer, kidney cancer, ovarian cancer, or melanoma. That is, the determination method of the present invention can also be interpreted as a method for assisting determination or diagnosis of lung cancer, kidney cancer, ovarian cancer, or melanoma.

[2. Apparatus and Computer Program for Cancer Determination]

The scope of the present invention also includes apparatuses for carrying out the cancer determination method of the present invention, as defined in the appended claims.

Such an apparatus is a cancer determination apparatus (hereinafter also simply referred to as "determination apparatus"). The scope of the present invention also includes a computer program for making a computer execute the above-described cancer determination method according to the present invention, as defined in the appended claims. Such a computer program is a computer program for determining lung cancer, kidney cancer, ovarian cancer, or melanoma.

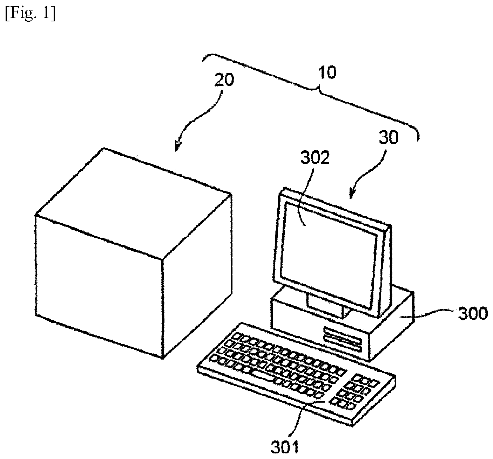

Hereinbelow, an example of the apparatus for carrying out the method of the present invention will be described with reference to the drawings. However, the present invention is not limited only to the embodiment shown in this example. Fig. 1 is a schematic diagram of a cancer determination apparatus. A determination apparatus 10 shown in Fig. 1 includes an immunoassay device 20 and a computer system 30 connected to the immunoassay device 20.

The type of immunoassay device is not particularly limited, and it can be appropriately selected according to the method for measuring a free protein marker. In the example shown in Fig. 1, the immunoassay device 20 is a commercially available automated immunoassay device capable of detecting a chemiluminescent signal generated by a sandwich ELISA method using magnetic particles on which a capture antibody is immobilized and an enzyme-labeled detection antibody. The immunoassay device 20 is not particularly limited as long as it can detect a signal based on the used labeling substance, and it can be appropriately selected according to the type of the labeling substance.

When a reagent containing magnetic particles on which a capture antibody is immobilized, a reagent containing an enzyme-labeled detection antibody and a liquid sample collected from a subject are set in the immunoassay device 20, the immunoassay device 20 performs an antigen-antibody reaction using each reagent, acquires a chemiluminescent signal as optical information based on the enzyme-labeled antibody specifically bound to a free protein marker, and transmits the obtained optical information to the computer system 30.

The computer system 30 includes a computer main body 300, an input unit 301, and a display unit 302 that displays specimen information, a determination result, and the like. The computer system 30 receives the optical information from the immunoassay device 20. Then, a processor of the computer system 30 executes a computer program for determining cancer, installed in a hard disk 313, based on the optical information. As shown in Fig. 1, the computer system 30 may be equipment separate from the immunoassay device 20, or may be equipment including the immunoassay device 20. In the latter case, the computer system 30 may itself be the determination apparatus 10. A commercially available automatic immunoassay device may be loaded with the computer program for determining cancer described above.

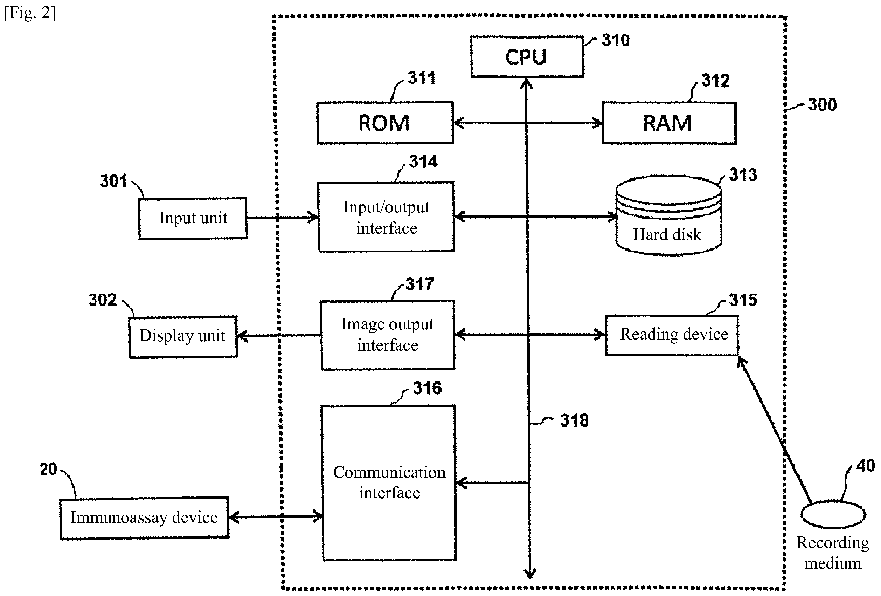

With reference to Fig. 2, the computer main body 300 includes a CPU (Central Processing Unit) 310, a ROM (Read Only Memory) 311, a RAM (Random Access Memory) 312, a hard disk 313, an input/output interface 314, a reading device 315, a communication interface 316, and an image output interface 317. The CPU 310, the ROM 311, the RAM 312, the hard disk 313, the input/output interface 314, the reading device 315, the communication interface 316 and the image output interface 317 are data-communicably connected by a bus 318. Further, the immunoassay device 20 is communicably connected to the computer system 30 via the communication interface 316.

The CPU 310 can execute a program stored in the ROM 311 or the hard disk 313 and a program loaded in the RAM 312. The CPU 310 calculates a measured value of a free protein marker, the CPU 310 reads a predetermined threshold value corresponding to each marker stored in the ROM 311 or the hard disk 313, and the CPU 310 determines whether or not the liquid sample is a sample collected from a subject suffering from lung cancer, kidney cancer, ovarian cancer, or melanoma. Alternatively, the CPU 310 may read the discriminant of multivariate analysis stored in the ROM 311 or the hard disk 313, and the CPU 310 may calculate a predicted value from the measured value of the free protein marker. Further, the CPU 310 may read a predetermined threshold value corresponding to the predicted value stored in the ROM 311 or the hard disk 313, and the CPU 310 may determine whether or not the liquid sample is a sample collected from a subject suffering from lung cancer, kidney cancer, ovarian cancer, or melanoma. The CPU 310 outputs the determination result and displays it on the display unit 302.

The ROM 311 includes a mask ROM, PROM, EPROM, EEPROM, and the like. In the ROM 311, a computer program executed by the CPU 310 and data used for executing the computer program are recorded as described above. In the ROM 311, data used for a determination flow to be described later, such as the predetermined threshold value and the discriminant of multivariate analysis, may be recorded.

The RAM 312 includes SRAM, DRAM, and the like. The RAM 312 is used for reading the program recorded in the ROM 311 and the hard disk 313. The RAM 312 is also used as a work area of the CPU 310 when executing these programs.

The hard disk 313 has installed therein an operating system to be executed by the CPU 310, a computer program such as an application program (the computer program for determining cancer), and data used for executing the computer program. In the hard disk 313, data used for a determination flow to be described later, such as the predetermined threshold value and the discriminant of multivariate analysis, may be recorded.

The reading device 315 is constituted of a flexible disk drive, a CD-ROM drive, a DVD-ROM drive, and the like. The reading device 315 can read a program or data recorded in a portable recording medium 40.

The input/output interface 314 includes, for example, a serial interface such as USB, IEEE1394 and RS-232C, a parallel interface such as SCSI, IDE and IEEE1284, and an analog interface including a D/A converter, an A/D converter and the like. The input unit 301 such as a keyboard and a mouse is connected to the input/output interface 314. An operator can input various commands to the computer main body 300 through the input unit 301.

The communication interface 316 is, for example, an Ethernet (registered trademark) interface or the like. The computer main body 300 can also transmit print data to a printer or the like through the communication interface 316.

The image output interface 317 is connected to the display unit 302 including an LCD, a CRT, and the like. As a result, the display unit 302 can output a video signal corresponding to the image data coming from the CPU 310. The display unit 302 displays an image (screen) according to the input video signal.

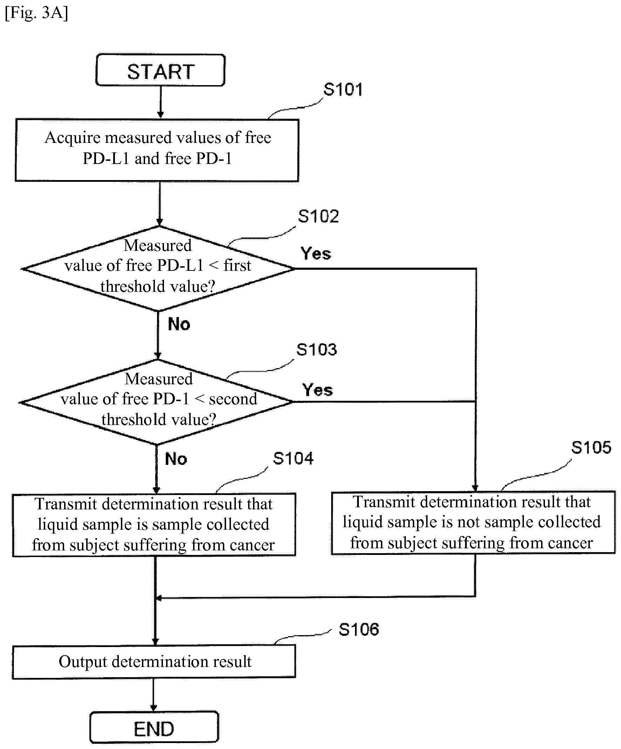

With reference to Fig. 3A, the cancer determination flow executed by the determination apparatus 10 will be described. Here, it will be described as an example a case where measured values of free PD-L1 and free PD-1 are acquired from a chemiluminescent signal generated by a sandwich ELISA method using magnetic particles on which a capture antibody is immobilized, and an enzyme-labeled detection antibody and a determination is made using the acquired measured value. However, the present invention is not limited only to this example.

In step S101, the CPU 310 acquires optical information (chemiluminescent signal) from the immunoassay device 20, the CPU 310 calculates the measured values of free PD-L1 and free PD-1 from the acquired optical information, and the CPU 310 stores them in the hard disk 313. In step S102, the CPU 310 compares the calculated measured value of free PD-L1 with the first threshold value stored in the hard disk 313. When the measured value of free PD-L1 is higher than the first threshold value or is equal to the first threshold value, the process proceeds to step S103. In step S103, the CPU 310 compares the calculated measured value of free PD-1 with the second threshold value stored in the hard disk 313. When the measured value of free PD-1 is higher than the second threshold value or is equal to the second threshold value, the process proceeds to step S104. In step S104, the CPU 310 stores in the hard disk 313 the determination result that the liquid sample is a sample collected from a subject suffering from lung cancer, kidney cancer, ovarian cancer, or melanoma.

On the other hand, when the measured value of free PD-L1 is lower than the first threshold value in step S102, the process proceeds to step S105. When the measured value of free PD-1 is lower than the second threshold value in step 103, the process proceeds to step S105. In step S105, the CPU 310 stores in the hard disk 313 the determination result that the liquid sample is not one collected from a subject suffering from lung cancer, kidney cancer, ovarian cancer, or melanoma. In step S106, the CPU 310 outputs the determination result, and displays it on the display unit 302, or makes a printer to print the determination result. Accordingly, it is possible to provide doctors and the like with information to assist the cancer determination of a subject, wherein the cancer is lung cancer, kidney cancer, ovarian cancer, or melanoma. In this example, the order of steps S102 and S103 can be changed.

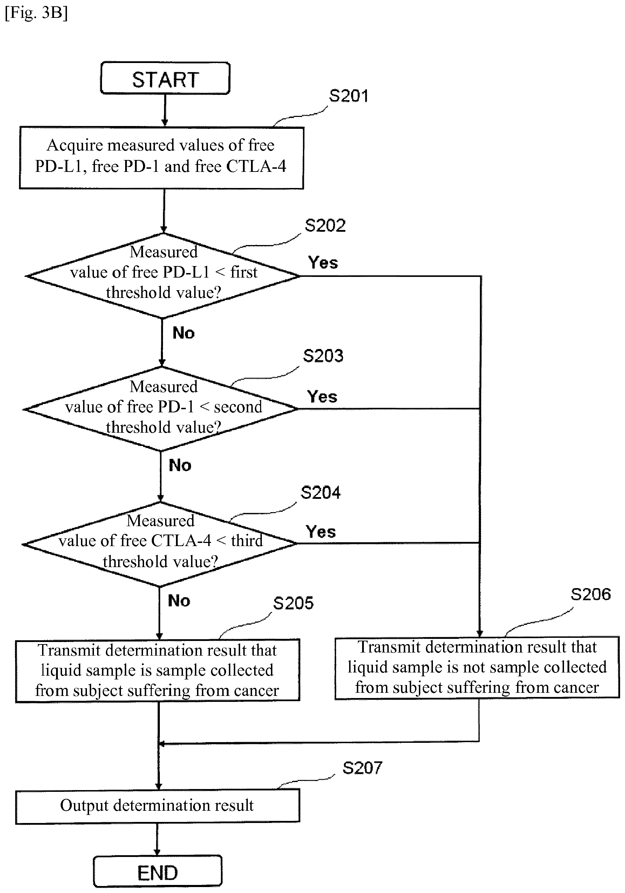

With reference to Fig. 3B, the cancer determination flow executed by the determination apparatus 10 will be described. In this example, it will be described as an example a case where the measured value of free CTLA-4 is further acquired, in addition to the measured values of free PD-L1 and free PD-1 and a determination is made using the acquired measured values. However, the present invention is not limited only to this example.

In step S201, the CPU 310 acquires optical information (chemiluminescent signal) from the immunoassay device 20, the CPU 310 calculates the measured values of free PD-L1, free PD-1 and free CTLA-4 from the acquired optical information, and the CPU 310 stores them in the hard disk 313. In step S202, the CPU 310 compares the calculated measured value of free PD-L1 with the first threshold value stored in the hard disk 313. When the measured value of free PD-L1 is higher than the first threshold value or is equal to the first threshold value, the process proceeds to step S203. In step S203, the CPU 310 compares the calculated measured value of free PD-1 with the second threshold value stored in the hard disk 313. When the measured value of free PD-1 is higher than the second threshold value or is equal to the second threshold value, the process proceeds to step S204. In step S204, the CPU 310 compares the calculated measured value of free CTLA-4 with the third threshold value stored in the hard disk 313. When the measured value of free CTLA-4 is higher than the third threshold value or is equal to the third threshold value, the process proceeds to step S205. In step S205, the CPU 310 stores in the hard disk 313 the determination result that the liquid sample is a sample collected from a subject suffering from lung cancer, kidney cancer, ovarian cancer, or melanoma.

On the other hand, when the measured value of free PD-L1 is lower than the first threshold value in step S202, the process proceeds to step S206. When the measured value of free PD-1 is lower than the second threshold value in step 203, the process proceeds to step S206. When the measured value of free CTLA-4 is lower than the third threshold value in step 204, the process proceeds to step S206. In step S206, the CPU 310 stores in the hard disk 313 the determination result that the liquid sample is not one collected from a subject suffering from lung cancer, kidney cancer, ovarian cancer, or melanoma. In step S207, the CPU 310 outputs the determination result, and displays it on the display unit 302, or makes a printer to print the determination result. Accordingly, it is possible to provide doctors and the like with information to assist the cancer determination of a subject, wherein the cancer is lung cancer, kidney cancer, ovarian cancer, or melanoma. In this example, the order of steps S202, S203 and S204 can be changed.

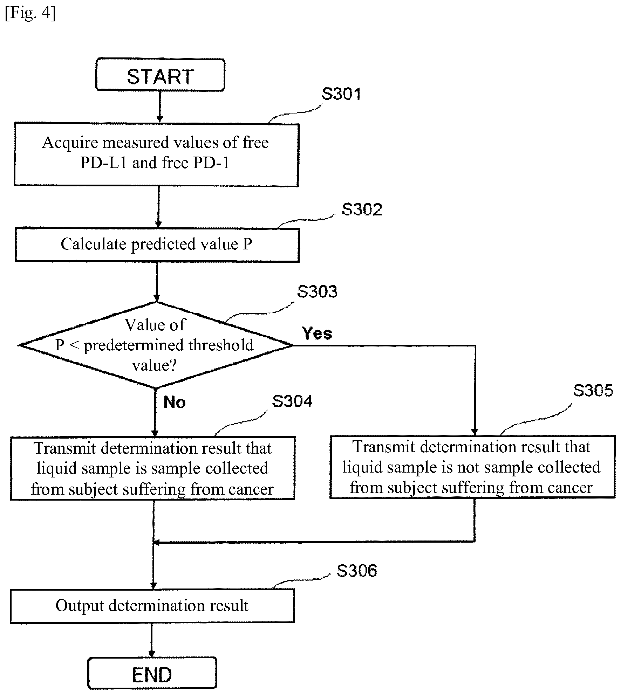

With reference to Fig. 4, the cancer determination flow executed by the determination apparatus 10 will be described. Here, it will be described as an example a case where a predicted value is acquired by a multiple logistic model from the acquired measured values of free PD-L1 and free PD-1 and a determination is made using the obtained predicted value. However, the present invention is not limited only to this example.

In step S301, the CPU 310 acquires optical information (chemiluminescent signal) from the immunoassay device 20, the CPU 310 calculates the measured values of free PD-L1 and free PD-1 from the acquired optical information, and the CPU 310 stores them in the hard disk 313. In step S302, the CPU 310 calculates a predicted value P based on the multiple logistic model according to the discriminant stored in the ROM 311 or the hard disk 313, from the measured values of free PD-L1 and free PD-1. In step S303, the CPU 310 compares the calculated predicted value P with a predetermined threshold value corresponding to the predicted value stored in the hard disk 313. When the predicted value P is higher than the predetermined threshold value or is equal to the predetermined threshold value, the process proceeds to step S304, and the CPU 310 stores in the hard disk 313 the determination result that the liquid sample is a sample collected from a subject suffering from lung cancer, kidney cancer, ovarian cancer, or melanoma. On the other hand, when the predicted value P is lower than the predetermined threshold value in step S303, the process proceeds to step S305, and the CPU 310 stores in the hard disk 313 the determination result that the liquid sample is not one collected from a subject suffering from lung cancer, kidney cancer, ovarian cancer, or melanoma. In step S306, the CPU 310 outputs the determination result, and displays it on the display unit 302, or makes a printer to print the determination result. Accordingly, it is possible to provide doctors and the like with information to assist the cancer determination of a subject, wherein the cancer is lung cancer, kidney cancer, ovarian cancer, or melanoma.

In this example, when the measured value of free CTLA-4 is further acquired, the CPU 310 may calculate a predicted value P from the measured values of free PD-L1, free PD-1 and free CTLA-4 in step S302.

Hereinbelow, the present invention will be described in detail by examples, but the present invention is not limited to these examples.

EXAMPLES

Example 1

In Example 1, a liquid sample derived from a healthy subject and a liquid sample derived from a cancer patient were discriminated based on concentration values of the free protein markers.

1. Liquid Sample

As a liquid sample, human plasma (containing EDTA) obtained from a healthy subject and a cancer patient was used. Plasma (50 specimens) of healthy subjects was obtained from BioreclamationIVT, and plasma (60 specimens) of cancer patients was obtained from Proteogenentec and BioreclamationIVT. The breakdown of the cancer patients are lung cancer patients (21 specimens), kidney cancer patients (18 specimens), melanoma patients (15 specimens), and ovarian cancer patients (6 specimens).

2. Measurement of Free Protein Markers

(2.1) Preparation of Reagents

(2.1.1) First Reagents (Biotin-Labeled Antibody Solutions)

• First reagent for capturing PD-L1

Anti-PD-L1 antibody (clone No. 27A2) was used as a capture antibody. This antibody was labeled with biotin using Biotin Labeling Kit-SH (Catalog No. LK10, manufactured by Chemical Dojin Co., Ltd.). Specific operations were carried out according to the manual attached to the kit. The obtained biotin-labeled anti-PD-L1 antibody was added to 100 mM MES (pH 6.5) to prepare a first reagent for capturing PD-L1 (antibody concentration 5 $\mu$g/ml).

• First reagent for capturing PD-1

Anti-PD-1 antibody (clone No. MIH4) was used as a capture antibody. This antibody was labeled with biotin in the same manner as described above. The obtained biotin-labeled anti-PD-1 antibody was added to 100 mM HEPES (pH 7.5) to prepare a first reagent for capturing PD-1 (antibody concentration 5 $\mu$g/ml).

• First reagent for capturing CTLA-4

Anti-CTLA-4 antibody (clone No. BNI3) was used as a capture antibody. This antibody was labeled with biotin in the same manner as described above. The obtained biotin-labeled anti-CTLA-4 antibody was added to 100 mM MES (pH 6.5) to prepare a first reagent for capturing CTLA-4 (antibody concentration 5 $\mu$g/ml).

(2.1.2) Second Reagent (Streptavidin-Bound Particle-Containing Liquid)

Magnetic particles on which streptavidin was immobilized on its surface (average particle size 2 $\mu$m; the amount of streptavidin per 1 g of the magnetic particles is 2.9 to 3.5 mg. hereinafter also referred to as "STA-bound magnetic particles") were washed 3 times with a 10 mM HEPES buffer solution (pH 7.5). The washed STA-bound magnetic particles were added to a 10 mM HEPES (pH 7.5) so as to have a streptavidin concentration of 18 to 22 $\mu$g/ml (a concentration of STA-bound magnetic particles of 0.48 to 0.52 mg/ml) to obtain an STA-bound particle-containing liquid.

(2.1.3) Third Reagents (Alkaline Phosphatase-Labeled Antibody Solutions)

• Third reagent for detecting PD-L1

Anti-PD-L1 antibody (clone No. 9L814) was used as a detection antibody. This antibody was labeled with alkaline phosphatase using Alkaline Phosphatase Labeling Kit-SH (Catalog No. LK13, manufactured by Chemical Dojin Co., Ltd.). Specific operations were carried out according to the manual attached to the kit. The obtained alkaline phosphatase-labeled anti-PD-L1 antibody was purified by gel filtration. This alkaline phosphatase-labeled anti-PD-L1 antibody was added to 100 mM MES (pH 6.5) so as to be diluted 75-fold to prepare a third reagent for detecting PD-L1.

• Third reagent for detecting PD-1

Anti-PD-1 antibody (clone No. PD1.3.1.3) was used as a detection antibody. This antibody was labeled with alkaline phosphatase and purified in the same manner as described above. The obtained alkaline phosphatase-labeled anti-PD-1 antibody was added to 100 mM HEPES (pH 7.5) so as to be diluted 75-fold to prepare a third reagent for detecting PD-1.

• Third reagent for detecting CTLA-4

Anti-CTLA-4 antibody (clone No. 14D3) was used as a detection antibody. This antibody was labeled with alkaline phosphatase and purified in the same manner as described above. The obtained alkaline phosphatase-labeled anti-CTLA-4 antibody was added to 100 mM MES (pH 6.5) so as to be diluted 75-fold to prepare a third reagent for detecting CTLA-4.

(2.1.4) Fourth Reagent (Buffer Solution for Measurement)

HISCL R4 reagent (manufactured by Sysmex Corporation) was used as a fourth reagent.

(2.1.5) Fifth Reagent (Substrate Solution)

HISCL R5 reagent (manufactured by Sysmex Corporation) containing CDP-Star (registered trademark) (Applied Biosystems) as an alkaline phosphatase chemiluminescent substrate was used as a fifth reagent.

(2.2) Measurement

Measurement of each free protein marker was performed with a fully automated immunoassay device HISCL-800 (manufactured by Sysmex Corporation) using the first to fifth reagents described above. This measurement is based on sandwich ELISA on magnetic particles. Specifically, plasma (20 $\mu$L) was added to the first reagent (50 $\mu$L) and mixed, then the second reagent (30 $\mu$L) was added and mixed. The magnetic particles in the obtained mixed solution were magnetically collected to remove the supernatant, and a HISCL washing solution (300 $\mu$L) was added to wash the magnetic particles. The supernatant was removed, and the third reagent (80 $\mu$L) was added to the magnetic particles and mixed. The magnetic particles in the obtained mixed solution were magnetically collected to remove the supernatant, and a HISCL washing solution (300 $\mu$L) was added to wash the magnetic particles. The supernatant was removed, and the fourth reagent (50 $\mu$L) and the fifth reagent (100 $\mu$L) were added to the magnetic particles and thoroughly mixed, and the chemiluminescence intensity was measured. The reaction time was 17 minutes throughout the entire process. The obtained chemiluminescence intensity was applied to the calibration curve to calculate the concentration of each free protein marker.

3. Determination and Result

A predicted value (P) of each specimen was calculated from concentration values of markers in liquid samples each derived from healthy subjects (n = 50) and cancer patients (n = 60), based on the multiple logistic model. Specifically, the predicted value was calculated from the following regression equation. This multiple logistic regression analysis was performed using StatFlex (manufactured by Artec Co., Ltd.).

$$\text{Regression equation: } P = 1/(1 + \exp(-x))$$

In the formula, "$\times$" varies depending on the marker used for determination. In Table 1, "$\times$"s corresponding to the markers used for determination are shown. In the table, [PD-L1], [PD-1] and [CTLA-4] are concentration values of free PD-L1, free PD-1 and free CTLA-4 in the liquid sample, respectively. Each coefficient in "$\times$" was calculated using data of all specimens (n = 110) including healthy subjects and cancer patients.

[Table 1]

| Marker | |
|---|---|
| PD-L1 | x = -4.4399 + 0.02837 $\times$ [PD-L1] |
| PD-1 | x = -3.3776 + 0.01925 $\times$ [PD-1] |
| CTLA-4 | x = -1.0339 + 0.57160 $\times$ [CTLA-4] |
| PD-L1 and PD-1 | x = -7.1206 + 0.02637 $\times$ [PD-L1] + 0.01959 $\times$ [PD-1] |
| PD-L1, PD-1 and CTLA-4 | x = -8.4135 + 0.02898 $\times$ [PD-L1] + 0.02105 $\times$ [PD-1] + 0.48802 $\times$ [CTLA-4] |

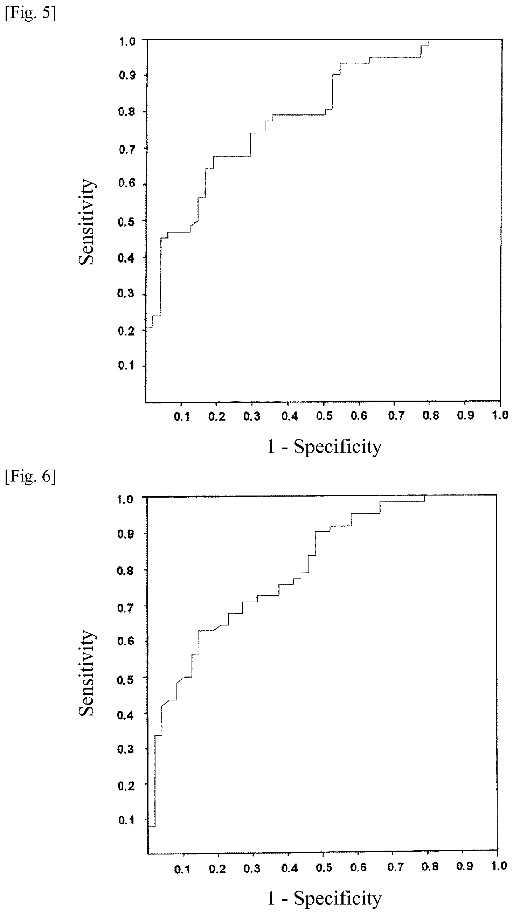

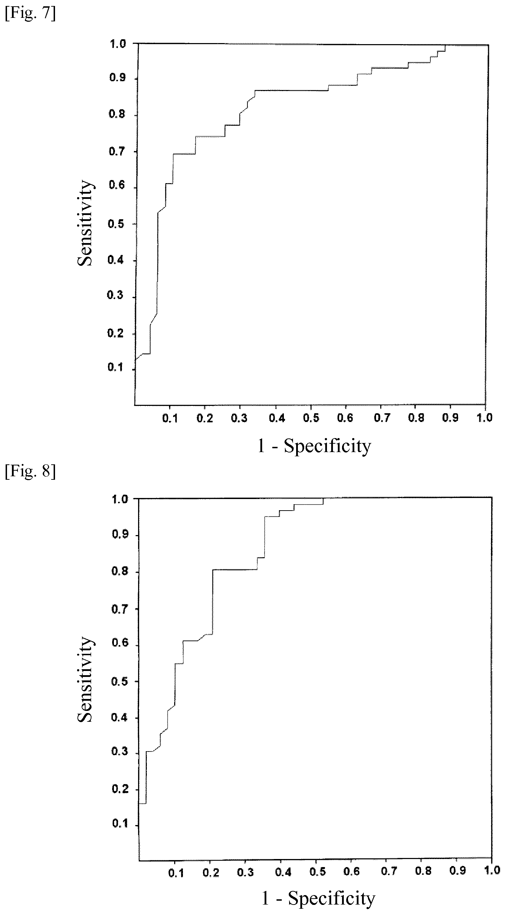

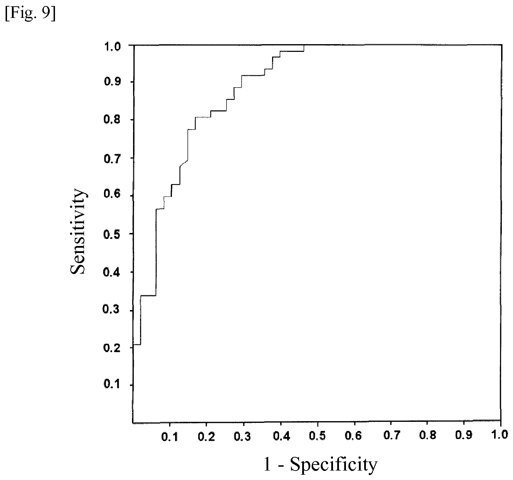

An ROC curve (Receiver Operating Characteristic curve) was created based on the calculated predicted value of each sample. The ROC curves corresponding to the markers used for the determination are shown in Figs. 5 to 9. Also, predetermined threshold values were determined from the ROC curves. Then, when the predicted value was higher than the predetermined threshold value or was equal to the predetermined threshold value, the liquid sample was determined to be a sample derived from a cancer patient, and when the predicted value was lower than the predetermined threshold value, the liquid sample was determined to be a sample derived from a healthy subject. Table 2 shows the area under the curve (AUC) of each ROC curve and the specificity when the sensitivity according to the above determination is about 90%. Incidentally, creation of ROC curves, calculation of threshold values, and calculations of sensitivity and specificity were carried out using StatFlex (manufactured by Artec Co., Ltd.).

[Table 2]

| Marker | AUC | Specificity (%) when sensitivity is about 90% |
|---|---|---|
| PD-L1 | 0.80242 | 47.92 |
| PD-1 | 0.79755 | 45.83 |
| CTLA-4 | 0.82241 | 37.50 |
| PD-L1 and PD-1 | 0.85534 | 64.58 |
| PD-L1, PD-1 and CTLA-4 | 0.89113 | 70.83 |

As shown in Table 2, the AUC was higher when using a combination of free PD-L1 and free PD-1, or a combination of free PD-L1, free PD-1 and free CTLA-4, than when singly using each of free PD-L1, free PD-1 and free CTLA-4 for determination. In addition, the specificity when the sensitivity according to the determination is about 90% is higher when using a combination of free PD-L1 and free PD-1, than when singly using each of free PD-L1 and free PD-1 for determination. This specificity increased by further combining free CTLA-4 with free PD-L1 and free PD-1. Therefore, it was shown that, based on concentration values of free PD-L1 and free PD-1 in a liquid sample, it is possible to discriminate whether or not the sample is a sample derived from a cancer patient with high accuracy. In addition, it was shown that the determination accuracy is further improved by further using concentration values of free CTLA-4 for determination.

REFERENCE SIGNS LIST

10: Determination apparatus
20: Immunoassay device
30: Computer system
40: Recording medium
300: Computer body
301: Input unit
302: Display unit
310: CPU
311: ROM
312: RAM
313: Hard disk
314: Input/output interface
315: Reading device
316: Communication interface
317: Image output interface
318: Bus

**Claims**

1. A method for determining cancer, the method comprising the steps of:

   measuring free PD-L1 (Programmed cell death-ligand 1) and free PD-1 (Programmed cell death-1) as free protein markers in a liquid sample collected from a subject, and
   determining whether or not the liquid sample is a sample collected from a subject suffering from cancer, wherein
   the cancer is lung cancer, kidney cancer, ovarian cancer, or melanoma, and
   in the determination step, measured values of the free protein markers acquired in the measurement step are compared with a predetermined threshold value corresponding to each of the markers, and whether or not the liquid sample is a sample collected from a subject suffering from the cancer is determined, based on the comparison result or
   in the determination step, a predicted value acquired by multivariate analysis from measured values of the free protein markers acquired in the measurement step is compared with a predetermined threshold value corresponding to the predicted value, and whether or not the liquid sample is a sample collected from a subject suffering from the cancer is determined, based on the comparison result.

2. The method according to claim 1, wherein

   in the measurement step, free CTLA-4 (Cytotoxic T lymphocyte antigen-4) is further measured as a free protein marker in the liquid sample, and
   in the determination step, whether or not the liquid sample is a sample collected from a subject suffering from the cancer is determined, based on measurement results of the free PD-L1, the free PD-1 and the free CTLA-4.

3. The method according to any one of claims 1 or 2, wherein the liquid sample is a blood sample.

4. The method according to any one of claims 1 to 3, wherein the multivariate analysis is logistic regression analysis.

5. A cancer determination apparatus comprising

   a computer containing a processor and a memory under control of the processor, wherein
   a computer program for making the computer execute the following steps of:

      acquiring measured values of free PD-L1 (Programmed cell death-ligand 1) and free PD-1 (Programmed cell death-1) as free protein markers in a liquid sample collected from a subject, and

determining whether or not the liquid sample is a sample collected from a subject suffering from cancer by comparing the measured values of the free protein markers with a predetermined threshold value corresponding to each of the markers, and determining whether or not the liquid sample is a sample collected from a subject suffering from the cancer based on the comparison result or

by comparing a predicted value acquired by multivariate analysis from measured values of the free protein markers with a predetermined threshold value corresponding to the predicted value, and determining whether or not the liquid sample is a sample collected from a subject suffering from the cancer based on the comparison result,

is recorded in the memory, and

wherein the cancer is lung cancer, kidney cancer, ovarian cancer, or melanoma.

6. The apparatus according to claim 5, wherein

in the step of acquiring measured values, a measured value of free CTLA-4 (Cytotoxic T lymphocyte antigen-4) is further acquired as a free protein marker in the liquid sample.

7. A computer program for determining cancer which is recorded on a computer readable medium, wherein

the computer program is a computer program for making the computer execute the following steps of:

acquiring measured values of free PD-L1 (Programmed cell death-ligand 1) and free PD-1 (Programmed cell death-1) as free protein markers in a liquid sample collected from a subject, and

determining whether or not the liquid sample is a sample collected from a subject suffering a cancer by comparing the measured values of the free protein markers with a predetermined threshold value corresponding to each of the markers, and determining whether or not the liquid sample is a sample collected from a subject suffering from the cancer based on the comparison result or

by comparing a predicted value acquired by multivariate analysis from measured values of the free protein markers with a predetermined threshold value corresponding to the predicted value, and determining whether or not the liquid sample is a sample collected from a subject suffering from the cancer based on the comparison result, and

wherein the cancer is lung cancer, kidney cancer, ovarian cancer, or melanoma.

8. The computer program according to claim 7, wherein

in the step of acquiring measured values, a measured value of free CTLA-4 (Cytotoxic T lymphocyte antigen-4) is further acquired as a free protein marker in the liquid sample.


## Patentansprüche

1. Verfahren zur Bestimmung von Krebs, wobei das Verfahren die folgenden Schritte umfasst:

Messen von freiem PD-L1 (programmierter Zelltod-Ligand 1) und freiem PD-1 (programmierter Zelltod-1) als freie Proteinmarker in einer flüssigen Probe, die einem Individuum entnommen ist, und

Bestimmen, ob die flüssige Probe eine Probe ist, die einem Individuum entnommen ist, der an Krebs leidet, oder nicht,

wobei

der Krebs Lungenkrebs, Nierenkrebs, Eierstockkrebs oder Melanom ist und im Bestimmungsschritt Messwerte der freien Proteinmarker, die im Messschritt erfasst werden, mit einem vorgegebenen Schwellenwert verglichen werden, der jedem der Marker entspricht, und basierend auf dem Vergleichsergebnis bestimmt wird, ob die flüssige Probe eine Probe ist, die einem Individuum entnommen ist, der an Krebs leidet, oder nicht, oder

im Bestimmungsschritt ein Vorhersagewert, der durch multivariate Analyse aus Messwerten der im Messschritt erfassten freien Proteinmarker erfasst wird, mit einem vorgegebenen Schwellenwert verglichen wird, der dem Vorhersagewert entspricht, und basierend auf dem Vergleichsergebnis bestimmt wird, ob die flüssige Probe eine Probe ist, die einem Individuum entnommen ist, der an Krebs leidet, oder nicht.

2. Verfahren nach Anspruch 1, wobei

im Messschritt ferner freies CTLA-4 (zytotoxisches T-Lymphozyten-Antigen-4) als freier Proteinmarker in der flüssigen Probe gemessen wird und

im Bestimmungswert basierend auf Messergebnissen des freien PD-L1, des freien PD-1 und des freien CTLA-4 bestimmt wird, ob die flüssige Probe eine Probe ist, die einem Individuum entnommen ist, der an Krebs leidet, oder nicht.

3. Verfahren nach einem der Ansprüche 1 oder 2, wobei die flüssige Probe eine Blutprobe ist.

4. Verfahren nach einem der Ansprüche 1 bis 3, wobei die multivariate Analyse eine logistische Regressionsanalyse ist.

5. Vorrichtung zur Bestimmung von Krebs, umfassend:
   einen Computer, der einen Prozessor und einen Speicher unter der Kontrolle des Prozessors enthält, wobei ein Computerprogramm zum Veranlassen des Computers zum Ausführen der folgenden Schritte:

   Erfassen von Messwerten von freiem PD-L1 (programmierter Zelltod-Ligand 1) und freiem PD-1 (programmierter Zelltod-1) als freie Proteinmarker in einer flüssigen Probe, die einem Individuum entnommen ist, und
   Bestimmen, ob die flüssige Probe eine Probe ist, die einem Individuum entnommen ist, der an Krebs leidet, oder nicht, durch Vergleichen der Messwerte der freien Proteinmarker mit einem vorgegebenen Schwellenwert, der jedem der Marker entspricht, und Bestimmen, ob die flüssige Probe eine Probe ist, die einem Individuum entnommen ist, der an Krebs leidet, oder nicht, basierend auf dem Vergleichsergebnis oder
   durch Vergleichen eines Vorhersagewerts, der durch multivariate Analyse aus Messwerten der freien Proteinmarker erfasst wird, mit einem vorgegebenen Schwellenwert, der dem Vorhersagewert entspricht, und Bestimmen, ob die flüssige Probe eine Probe ist, die einem Individuum entnommen ist, der an Krebs leidet, oder nicht, basierend auf dem Vergleichsergebnis.
   im Speicher aufgezeichnet ist, und
   wobei der Krebs Lungenkrebs, Nierenkrebs, Eierstockkrebs oder Melanom ist.

6. Vorrichtung nach Anspruch 5, wobei:
   im Schritt des Erfassens von Messwerten ferner ein Messwert von freiem CTLA-4 (zytotoxisches T-Lymphozyten-Antigen-4) als freier Proteinmarker in der flüssigen Probe erfasst wird.

7. Computerprogramm zum Bestimmen von Krebs, das auf einem computerlesbaren Medium aufgezeichnet ist, wobei
   das Computerprogramm ein Computerprogramm zum Veranlassen des Computers zum Ausführen der folgenden Schritte ist:

   Erfassen von Messwerten von freiem PD-L1 (programmierter Zelltod-Ligand 1) und freiem PD-1 (programmierter Zelltod-1) als freie Proteinmarker in einer flüssigen Probe, die einem Individuum entnommen ist, und
   Bestimmen, ob die flüssige Probe eine Probe ist, die einem Individuum entnommen ist, der an Krebs leidet, oder nicht, durch Vergleichen der Messwerte der freien Proteinmarker mit einem vorgegebenen Schwellenwert, der jedem der Marker entspricht, und Bestimmen, ob die flüssige Probe eine Probe ist, die einem Individuum entnommen ist, der an Krebs leidet, oder nicht, basierend auf dem Vergleichsergebnis oder
   durch Vergleichen eines Vorhersagewerts, der durch multivariate Analyse aus Messwerten der freien Proteinmarker erfasst wird, mit einem vorgegebenen Schwellenwert, der dem Vorhersagewert entspricht, und Bestimmen, ob die flüssige Probe eine Probe ist, die einem Individuum entnommen ist, der an Krebs leidet, oder nicht, basierend auf dem Vergleichsergebnis und wobei der Krebs Lungenkrebs, Nierenkrebs, Eierstockkrebs oder Melanom ist.

8. Computerprogramm nach Anspruch 7, wobei im Schritt des Erfassens von Messwerten ferner ein Messwert von freiem CTLA-4 (zytotoxisches T-Lymphozyten-Antigen-4) als freier Proteinmarker in der flüssigen Probe erfasst wird.

**Revendications**

1. Procédé de détermination d'un cancer, le procédé comprenant les étapes suivantes :

   la mesure de PD-L1 libre (ligand 1 de mort cellulaire programmée) et de PD-1 libre (mort cellulaire programmée 1) en tant que marqueurs protéiques libres dans un échantillon de liquide prélevé sur un sujet, et
   la détermination si l'échantillon de liquide est ou non un échantillon prélevé sur un sujet souffrant d'un cancer, le cancer étant un cancer du poumon, un cancer du rein, un cancer de l'ovaire ou un mélanome, et dans l'étape de détermination, les valeurs mesurées des marqueurs protéiques libres acquises dans l'étape de mesure étant comparées à une valeur seuil prédéterminée correspondant à chacun des marqueurs, et le fait que l'échantillon liquide est ou non un échantillon prélevé sur un sujet souffrant du cancer, étant déterminé sur la base du résultat de la comparaison ou

dans l'étape de détermination, une valeur prédite obtenue par analyse multivariée à partir des valeurs mesurées des marqueurs de protéines libres acquises dans l'étape de mesure étant comparée à une valeur seuil prédéterminée correspondant à la valeur prédite, et le fait que l'échantillon de liquide est ou non un échantillon prélevé sur un sujet souffrant d'un cancer étant déterminé sur la base du résultat de la comparaison.

2. Procédé selon la revendication 1,

dans l'étape de mesure, le CTLA-4 libre (antigène 4 du lymphocyte T cytotoxique) étant en outre mesuré en tant que marqueur protéique libre dans l'échantillon liquide, et
dans l'étape de détermination, le fait que l'échantillon liquide est ou non un échantillon prélevé sur un sujet atteint d'un cancer, étant déterminé sur la base des résultats de mesure du PD-L1 libre, du PD-1 libre et du CTLA-4 libre.

3. Procédé selon l'une quelconque des revendications 1 ou 2, l'échantillon liquide étant un échantillon de sang.

4. Procédé selon l'une quelconque des revendications 1 à 3, l'analyse multivariée étant une analyse de régression logistique.

5. Appareil de détermination d'un cancer comprenant :

un ordinateur contenant un processeur et une mémoire commandée par le processeur,
un programme informatique permettant à l'ordinateur d'exécuter les étapes suivantes :

acquisition des valeurs mesurées de PD-L1 libre (ligand 1 de mort cellulaire programmée) et PD-1 libre (mort cellulaire programmée 1) en tant que marqueurs protéiques libres dans un échantillon liquide prélevé sur un sujet, et
détermination si l'échantillon de liquide est ou non un échantillon prélevé sur un sujet souffrant d'un cancer en comparant les valeurs mesurées des marqueurs protéiques libres à une valeur seuil prédéterminée correspondant à chacun des marqueurs, et détermination si l'échantillon de liquide est ou non un échantillon prélevé sur un sujet souffrant d'un cancer sur la base du résultat de la comparaison ou
comparaison d'une valeur prédite obtenue par analyse multivariée à partir des valeurs mesurées des marqueurs protéiques libres avec une valeur seuil prédéterminée correspondant à la valeur prédite, et détermination si l'échantillon de liquide est ou non un échantillon prélevé sur un sujet souffrant d'un cancer sur la base du résultat de la comparaison,
est enregistré dans la mémoire, et
le cancer étant un cancer du poumon, un cancer du rein, un cancer de l'ovaire ou un mélanome.

6. Appareil selon la revendication 5,
dans l'étape d'acquisition des valeurs mesurées, une valeur mesurée de CTLA-4 (antigène 4 du lymphocyte T cytotoxique) libre étant en outre acquise en tant que marqueur protéique libre dans l'échantillon liquide.

7. Programme informatique de détermination d'un cancer enregistré sur un support lisible par ordinateur,
le programme informatique étant un programme informatique permettant à l'ordinateur d'exécuter les étapes suivantes :

acquisition des valeurs mesurées de PD-L1 libre (ligand 1 de mort cellulaire programmée) et PD-1 libre (mort cellulaire programmée 1) en tant que marqueurs protéiques libres dans un échantillon liquide prélevé sur un sujet, et
détermination si l'échantillon de liquide est ou non un échantillon prélevé sur un sujet atteint d'un cancer en comparant les valeurs mesurées des marqueurs protéiques libres à une valeur seuil prédéterminée correspondant à chacun des marqueurs, et détermination si l'échantillon de liquide est ou non un échantillon prélevé sur un sujet atteint d'un cancer sur la base du résultat de la comparaison ou
comparaison d'une valeur prédite obtenue par analyse multivariée à partir des valeurs mesurées des marqueurs protéiques libres avec une valeur seuil prédéterminée correspondant à la valeur prédite, et détermination si l'échantillon de liquide est ou non un échantillon prélevé sur un sujet atteint d'un cancer sur la base du résultat de la comparaison, et le cancer étant un cancer du poumon, un cancer du rein, un cancer de l'ovaire ou un mélanome.

8. Programme informatique selon la revendication 7, dans l'étape d'acquisition des valeurs mesurées, une valeur mesurée de CTLA-4 (antigène 4 du lymphocyte T cytotoxique) libre étant en outre acquise en tant que marqueur protéique libre dans l'échantillon liquide.

[Fig. 1]

[Fig. 2]

EP 3 399 314 B1

[Fig. 3A]

START

S101

Acquire measured values of free
PD-L1 and free PD-1

S102

Measured
value of free PD-L1 < first
threshold value?

Yes

No

S103

Measured
value of free PD-1 < second
threshold value?

Yes

No

S104

S105

Transmit determination result that
liquid sample is sample collected
from subject suffering from cancer

Transmit determination result that
liquid sample is not sample collected
from subject suffering from cancer

S106

Output determination result

END

[Fig. 3B]

```
                    ┌──────────────┐
                    │    START     │
                    └──────┬───────┘
                           │              S201
          ┌────────────────▼────────────────┐
          │  Acquire measured values of free │
          │  PD-L1, free PD-1 and free CTLA-4│
          └────────────────┬────────────────┘
                           │              S202
                        ╱──▼──╲              Yes
                    ╱ Measured ╲──────────────────────────┐
                   ⟨ value of free PD-L1 < first ⟩          │
                    ╲  threshold value?  ╱                  │
                        ╲──┬──╱                             │
                          No                                │
                           │              S203              │
                        ╱──▼──╲              Yes            │
                    ╱ Measured ╲─────────────────┐          │
                   ⟨ value of free PD-1 < second ⟩│          │
                    ╲  threshold value?  ╱        │          │
                        ╲──┬──╱                   │          │
                          No                      │          │
                           │              S204    │          │
                        ╱──▼──╲              Yes  │          │
                    ╱ Measured ╲───────────┐      │          │
                   ⟨ value of free CTLA-4 < third⟩│      │          │
                    ╲  threshold value?  ╱  │      │          │
                        ╲──┬──╱             │      │          │
                          No      S205      │      │          │
          ┌────────────────▼────────────────┐  ┌──▼──────▼──────▼──┐
          │ Transmit determination result that│  │ Transmit determination result that
          │ liquid sample is sample collected │  │ liquid sample is not sample collected
          │ from subject suffering from cancer│  │ from subject suffering from cancer
          └────────────────┬────────────────┘  └─────────┬─────────┘ S206
                           │◄───────────────────────────────┘
                           │              S207
          ┌────────────────▼────────────────┐
          │   Output determination result    │
          └────────────────┬────────────────┘
                    ┌──────▼───────┐
                    │     END      │
                    └──────────────┘
```

[Fig. 4]

```
                    ┌──────────────┐
                    │    START     │
                    └──────────────┘
                           │              S301
                           ▼
          ┌────────────────────────────────┐
          │   Acquire measured values of free │
          │      PD-L1 and free PD-1         │
          └────────────────────────────────┘
                           │              S302
                           ▼
          ┌────────────────────────────────┐
          │     Calculate predicted value P  │
          └────────────────────────────────┘
                           │                    S303
                           ▼
                   ╱───────────────╲
                  ╱    Value of      ╲        Yes
          ╱──────  P < predetermined threshold ──────────────────┐
                  ╲       value?      ╱                           │
                   ╲───────────────╱                              │
                           │ No            S304          S305     │
                           ▼                                      ▼
      ┌──────────────────────────────┐   ┌──────────────────────────────┐
      │  Transmit determination result that │ │ Transmit determination result that │
      │  liquid sample is sample collected │ │ liquid sample is not sample collected │
      │  from subject suffering from cancer│ │ from subject suffering from cancer │
      └──────────────────────────────┘   └──────────────────────────────┘
                           │                                      │
                           │◄─────────────────────────────────────┘
                           │                 S306
                           ▼
          ┌────────────────────────────────┐
          │    Output determination result   │
          └────────────────────────────────┘
                           │
                           ▼
                    ┌──────────────┐
                    │     END      │
                    └──────────────┘
```

[Fig. 5]

[Fig. 6]

[Fig. 7]

1 - Specificity

[Fig. 8]

1 - Specificity

[Fig. 9]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H01254868 A

### Non-patent literature cited in the description

- **AGATA Y. et al.** Expression of the PD-1 antigen on the surface of stimulated mouse T and B lymphocytes. *Int. Immunol.,* 1996, vol. 8, 765-772

- **ZHANG et al.** Circulating PD-L1 in NSCLC patients and the correlation between the level of PD-L1 expression and the clinical characteristics. *Thoracic Cancer,* 2015, vol. 6, 534-538